# EUROPEAN PATENT APPLICATION

(11) **EP 2 255 805 A1**
(43) Date of publication of application: **01.12.2010**
(21) Application number: 10174063.7
(22) Date of filing: 21.05.2007
(51) Int. Cl.: A61K 31/4196, A61P 3/12, A61P 43/00

(54) **Deferasirox for the treatment of hereditary hemochromatosis**

(30) Priority: 04.08.2006 US 835658 P; 24.05.2006 US 803049 P; 23.05.2006 US 747974 P
(62) Divisional of application: 07840190.8
(71) Applicant: Novartis AG, 4056 Basel (CH); Novartis Pharma AG, 1230 Vienna (AT)
(72) Inventor: Nick, Hanspeter, 4202, Duggingen (CH); Rojkaer, Lisa, Princeton, NJ 08540 (US); Bodner, Janet, Bernardsville, NJ 07924 (US); Marks, Peter Wayne, Woodbridge, CT 06525-1913 (US)
(74) Representative: Roth, Peter Richard

(57) **Abstract**

The present invention relates to the use of Compound I of the following formula 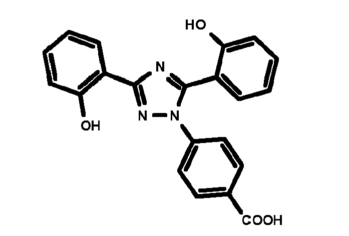 in the treatment of iron overload resulting from hereditary hemochromatosis in patient in need of such a treatment and to a commercial package comprising Compound I together with instructions for the treatment of iron overload in hereditary hemochromatosis patients.

## Description

The present invention relates to the use of Compound I in the treatment of hereditary hemochromatosis and to a commercial package comprising Compound I together with instructions for the treatment of hereditary hemochromatosis. The present invention relates to the use of Compound I for the treatment of iron overload in hereditary hemochromatosis patients.

### Background of the invention

Hereditary hemochromatosis, abbreviated as HH, is the most common inherited single-gene disorder in the Caucasian population. HFE gene is the name of the hemochromatosis gene. The HFE gene mutation is most frequently associated with hereditary hemochromatosis, and is located on the short arm of chromosome 6. Approximately one in 250 to 300 white persons is homozygous for the C282Y HFE gene mutation and at least one in 10 persons is a carrier, i.e. the mutation replaces cysteine with tyrosine at position 282 in the protein's chain of amino acids, also written as C282Y or Cys282Tyr. Another common mutation in the HFE gene that can lead to iron overload is the H63D mutation, i.e. the mutation replaces histidine with aspartic acid at position 63, written as H63D or His63Asp. The risk of iron overload is higher in person who are homozygous for the C282Y mutation, compared to C282Y/H63D compound heterozygotes or H63D homozygotes. Another mutation in the HFE gene, S65C accounts for nearly 8% of hemochromatosis mutations that are neither C282Y nor H63D. The exact function of the HFE gene product is not known however it appears to take part in the regulation of hepcidin expression.

HJV, Juvenile hemochromatosis is due to mutations in the HJV gene coding for hemojuvelin and HAMP gene coding for hepcidin. With juvenile hemochromatosis, iron accumulation begins early in life, and symptoms are present before the age of 30 years. Men and women are affected equally. The signs and symptoms of juvenile hemochromatosis are more severe than those of the HFE type and complications involving endocrine glands and, clinically most relevant, the heart. The disease, Juvenile hemochromatosis due to mutations of HJV and HAMP is very rare but it has been reported in several different ethnic groups. Mutations in the HAMP and HFE2 genes cause HJV. The HAMP and HFE2 genes appear to play a role in regulating iron absorption during digestion. The HAMP gene instructs cells to manufacture a small protein called hepcidin, which was originally identified as having antimicrobial properties. Recent evidence indicates that hepcidin levels are important in determining the amount of iron absorbed from the diet. Low levels of hepcidin, e.g. due to HAMP mutation, increases the iron influx from the intestinal mucosa. Hemojuvelin acts upstream of hepcidin and co-determines the amount of hepcidin expression. Researchers recently discovered that hepcidin level plays a role in maintaining iron balance in humans.

Generally, the various types of hereditary hemochromatosis are characterized by increased intestinal absorption of iron, which leads to deposition of iron in the body, i.e. in multiple organs such as liver, pancreas, heart and other organs. Excess iron deposition, if left untreated, causes tissue damage and fibrosis with irreversible damage to various organs, e.g. iron overloaded organs, e.g. endocrine dysfunction or hepatic cirrhosis.

Initiation of treatment of hereditary hemochromatosis is indicated for patients with evidence of iron overload based on elevated iron parameters or markers, e.g. serum ferritin and transferrin saturation. Phlebotomy is the current standard of care because it is simple, effective and safe. Phlebotomy entails removal of up to two units of blood, e.g. 800 to 1000 ml corresponding to approximately 500 mg of iron per week, for information a unit of blood, e.g. approximately 500 ml removes about 250 mg iron. The aim is to remove excess body iron and lower the ferritin value to 50 mcg/L (microgram per liter) or less. This phase, which takes to 1-3 years rarely more, corresponds to the so called "induction phase". Once iron depletion is accomplished, most patients require four to eight phlebotomies per year to keep the serum ferritin concentration below 50 mcg/L, this corresponds to the so called "maintenance phase".

However 5 to 10% of patients are ineligible for, inadequately treated by, e.g. due to poor compliance, or unwilling to have phlebotomies due to poor venous access, anemia, congestive heart failure/ left ventricular dysfunction, or "needlephobia".

An alternative to phlebotomy is the use of iron chelation. Iron chelator can bind iron and promote its secretion. During this procedure, a drug that binds to excess free iron is administered.

Therefore, there is a need to provide an alternative treatment for hereditary hemochromatosis patients, e.g. for hemochromatosis patients unwilling or unable to undergo a phlebotomy regimen. As the amount of iron removed per phlebotomy is limited, and the number of phlebotomies an individual is able to tolerate is also limited, there is also a need for an alternative treatment to be used in combination with standard care, i.e. phlebotomy, to shorten the duration of iron removal in patients with modestly elevated ferritin levels, in order to prevent progression of end-organ damage.

The treatment of iron overload by subcutaneous desferrioxamine (abbreviated as DFO), i.e. Desferal®, was studied in 3 patients with HH in which phlebotomy was not, or transiently not possible because of their serious clinical conditions or the lack of peripheral venous access. DFO treatment was shown to be as effective as weekly phlebotomy, e.g. 500 ml (Nielsen P. et al, Br. J. Haematol 2003, 123(5):952-3). However available data reporting the use of desferrioxamine in this patient population is very limited.

Moreover, despite the merits of DFO, DFO cannot be administered orally and its half life within the bloodstream is short. DFO is administered via intravenous or subcutaneous infusion 8-12h /day at least 5 days per week and leads to poor patient compliance.

Compound of formula I as disclosed below is known to be used in the treatment of iron overload in blood-transfused patients, e.g. in beta-thalassemia patients. However, no or very little information is available on the safety of Compound I at low ferritin levels, e.g. ferritin levels below 500 mcg/L, e.g. 100 mcg/L, e.g. 50 mcg/L. Therefore, there was a need to determine the safety of Compound I at such low ferritin values, e.g. in order to determine a suitable treatment schedule for the use of Compound I in the removal of iron in hereditary hemochromatosis patients. Surprisingly, the inventors have found that Compound I is suitable and safe for hereditary hemochromatosis patients.

### Description of the drawings:

Figure 1A-D shows organ iron content verses time in weeks for the liver, heart, spleen and pancreas.

### Detailed Description of the Invention:

Compound I is a 3, 5-diphenyl-1,2,4-triazoles of the following formula: 4-[3,5-bis(2-hydroxyphenyl)-[1,2,4]triazol-1-yl]benzoic acid and can be administered orally.

Compound I has the following formula:

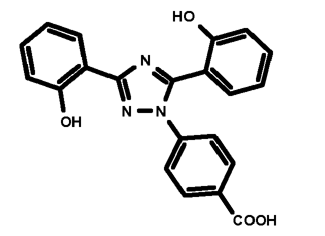

Compound I in the free acid form, salts thereof and its crystalline forms are disclosed in the International Patent Publication WO 97/49395, which is hereby incorporated by reference (published on December 31, 1997).

Compound I and its process of preparation is described, e.g. in US 6,465,504 B1.

Pharmaceutical preparation comprising Compound I are described in the International Patent Application WO 97/49395.

However US 6,465,504 does not disclose that Compound I is suitable to treat the iron overload due to hereditary hemochromatosis. US 6, 465, 504 does not disclose either the particular dosage, nor the treatment schedule as described herein after.

Within the scope of the present invention by "treatment of hereditary hemochromatosis" is meant the control of the body iron balance of the HH patient.

An HH patient is to be understood to be a patient having hereditary hemochromatosis or juvenile hemochromatosis. Hereditary hemochromatosis and juvenile hemochromatosis being defined as in the background of the invention.

The treatment of hereditary hemochromatosis can be performed according to an intermittent regimen, i.e. Compound I is administered until the serum ferritin reaches a defined value, e.g. of about 100 mcg/L or below, e.g. of 100 mcg/L or below, e.g. of about 50 mcg/L and the treatment is resumed as soon as the serum ferritin level exceeds another defined value, e.g. of about 300 mcg/L or above, e.g. of 300 mcg/L or above, e.g. of about 200 mcg/L or above , e.g. of about 200 mcg/L or above for female patients, e.g. of about 300 mcg/L or above for male patients.

The present invention pertains to the treatment of iron overload in hereditary hemochromatosis patients according to the intermittent treatment described above.

The treatment of hereditary hemochromatosis can be performed in two consecutive steps according to a second regimen, i.e. in a first phase, e.g. called the induction phase, Compound I is administered at a daily dose of 5 to 40 mg/kg of body weight, e.g. 5 to 20 mg/kg of body weight, e.g. 20 to 40 mg/kg body weight, with or without concomitant phlebotomy, e.g. until the serum ferritin level reaches a value of about 100 mcg/L or below, e.g. of 100 mcg/L, e.g. of about 50 mcg/L or below. The daily dose of Compound I administered during the induction phase can be determined based on the measured serum ferritin level of the HH patient. For example, for iron overloaded patients, e.g. with a serum ferritin level above 1500 mcg/L, or e.g. with a serum ferritin level above 200 mcg/L for the female patient and above 300 mcg/L for the male patient, concomitant administration of Compound I, e.g. at an appropriate dosage and phlebotomy can be performed. This combination has surprising advantage benefit also for e.g. hereditary hemochromatosis patients suffering from arthropathy. For example for patients having a serum ferritin level of about or above 300 mcg/L, e.g. of about or above 200 mcg/L for women, e.g. or about or above 300 mcg/L for men, the patients can be administered Compound I, e.g. at a daily dose of 5 to 20 mg/kg of body weight, e.g. until the serum ferritin level falls below, e.g. 100 mcg/L, e.g. 50 mcg/L. The patient is switched to the maintenance phase, e.g. at a daily or intermittent dose of 1 to 20 mg/kg of body weight, e.g. of 2 to 20 mg/ kg of body weight, e.g. 5 to 20 mg/day per kg of body weight, e.g. as soon as the patient's serum ferritin level exceeds a defined value, e.g. of about 300 mcg/L , e.g. of 300 mcg/L, e.g. of about 200 mcg/L , e.g. 200 mcg/L for female patients, e.g. of about 300 mcg/L for male patients. Alternatively, after the induction phase, the patient is switched to the intermittent regimen.

Alternatively, after the induction phase is completed, the patient iron overload is managed by phlebotomy, e.g. 3 or 4 times per year.
Alternatively, the patient is treated in the maintenance phase following an induction phase using phlebotomy only.

For the treatment of hereditary hemochromatosis, the dosage of Compound I within the maintenance phase can depend on various factors, such as for example, the age, the body weight and/or the individual condition of the warm-blooded animal, e.g. human to be treated.

The oral daily dose of Compound I to be administered to a patient with hereditary hemochromatosis during the maintenance phase is from, e.g. 1 to 10 mg/kg of body weight per day, e.g. 1 to 5 mg/ kg of body weight per day, in particular from 2 to 4 mg/kg of body weight per day. For a warm-blooded animal, e.g. a human having a body weight of approximately 40 kg, the oral daily dose of Compound I is approximately of from 40 to 200 mg/day for the maintenance phase of hereditary hemochromatosis.

Compound I has a plasma half life of approximately 8 to 16 hours, e.g. 12 to 13 hours within the human body. Therefore, alternatively, the dose of Compound I to be administered can be taken every other day, e.g. for a person of a body weight of 40 kg, one tablet of 80 to 400 mg can be taken every other day, e.g. on for two consecutive weeks on days 1, 3, 5, 7 for the first week and on days 2,4, and 6 for the subsequent week. Alternatively the dose of Compound I as mentioned above can daily divided, e.g. the daily dose as mentioned above can be split and given as two administrations.

The present invention relates to the use of for the preparation of a medicament for the control of the body iron content in patients having HH.

In one embodiment, the invention relates to the use of Compound I for the preparation of a medicament for the treatment of HH wherein Compound I is administered :
(a) during the induction phase at a daily dose of 20 to 40 mg/kg body weight with or without concomitant phlebotomy, then
(b) during the maintenance phase at a daily dose of, e.g. 1 to 10 mg/kg of body weight, e.g. 5 to 20 mg/kg of body weight.

In one embodiment, the invention relates to the use of Compound I for the preparation of a medicament for the treatment of HH wherein Compound I is administered :
(a) during the induction phase at a daily dose of 20 to 40 mg/kg body weight with or without concomitant phlebotomy, then
(b) during the maintenance phase at an intermittent dose of, e.g. 1 to 10 mg/kg of body weight, e.g. 5 to 20 mg/kg of body weight administered for 1, 2, 3 or 4 weeks.

In one embodiment, the invention relates to the use of Compound I for the preparation of a medicament for the treatment of HH wherein Compound I is administered :
(a) during the induction phase at a daily dose of 20 to 40 mg/kg body weight with or without concomitant phlebotomy, then
(b) during the maintenance phase at a fixed dose of, e.g. comprising and including 500 mg to 2 g per day, e.g. 500 mg, 1 g, or 2 g, administered daily for, e.g. 1 or 2 weeks every, e.g. 3 to 4 months, e.g. 3 or 4 months.

In one embodiment, the invention relates to the use of Compound I for the preparation of a medicament for the treatment of HH wherein Compound I is administered :
(a) during the induction phase at a daily dose of 20 to 40 mg/kg body weight with or without concomitant phlebotomy, then
(b) during the maintenance phase at a dose of Compound I as needed based upon e.g. the ferritin level of the patient blood and/or e.g. the patient body weight.

In one embodiment, the invention relates to the use of Compound I for the preparation of a medicament for the treatment of HH wherein Compound I is administered :
(a) during the induction phase at a daily dose of 5 to 20 mg/kg body weight with or without concomitant phlebotomy, then
(b) during the maintenance phase at a daily dose of, e.g. 1 to 10 mg/kg of body weight, e.g. 1 to 5 mg/kg of body weight, e.g. 5 to 20 mg/kg of body weight.

In a further embodiment, the invention relates to the use of Compound I for the preparation of a medicament for the treatment of HH wherein Compound I is administered intermittently.

The present invention pertains to the use of Compound I for the preparation of a medicament for the treatment of HH wherein Compound I is administered at a daily dose of 5 to 20mg/kg of body weight with or without concomitant phlebotomy until the serum ferritin level is below 100 mcg/L.
The present invention pertains to the use of Compound I for the preparation of a medicament for the treatment of HH wherein Compound I is administered at a daily dose of 5 to 20mg/kg of body weight with or without concomitant phlebotomy until the serum ferritin level reaches a level of 50 mcg/L.

By "intermittently" is meant that the administration of Compound I starts as soon as the serum ferritin level reaches a value of about or equal to 200 to 300 mcg/L , e.g. 300 mcg/L , e.g. 200 mcg/L , e.g. 200 mcg/L for a female patient, e.g. 300 mcg/L for a male patient and that the treatment is held when the serum ferritin level reaches the value of about 50-100 mcg/L or below, e.g. of about 100 mcg/L , e.g. of about 50 mcg/L. The treatment is resumed when the serum ferritin level reaches again the value of about or equal to 300 mcg/L, e.g. when the serum ferritin level exceeds a value, e.g. of 300 mcg/L , e.g. of 200 mcg/L, e.g. of 200 mcg/L for a female patient, e.g. of 300 mcg/L for a male patient.

In one embodiment of the invention, the invention relates to the use of Compound I for the preparation of a medicament for the treatment of HH wherein Compound I is administered intermittently at a dose of 5, 10, 15 or 20 mg/kg of body weight per day.

In a further embodiment the invention pertains to a commercial package comprising Compound I together with instructions for the administration of Compound I to hereditary hemochromatosis patients.

The present invention pertains to a:
1. Method of treating iron overload resulting from hereditary hemochromatosis comprising the administering to a patient in need of such a treatment a therapeutically effective amount of Compound I.
2. Method of treating hereditary hemochromatosis which causes iron overload in the human body comprising the administering to a patient in need of such a treatment a therapeutically effective amount of Compound I.
3. Method according to 1 or 2 above wherein the hereditary hemochromatosis is due to the mutation C282Y or H63D.
4. Method according to 1 or 2 above wherein Compound I is administered to a patient in need of such a treatment at a daily dose of about 1 to 20 mg of Compound I /kg of body weight , e.g. 1 to 10 mg/kg of body weight, e.g. 5 to about 20 mg of Compound I /kg of body weight.
5. Method according to 1 or 2 or 3 above wherein the treatment is discontinued when the serum ferritin level is of about 100 mcg/L or below, e.g. of about 50 to 100 mcg/L or below.
6. Method according to 1, 2 or 3 above wherein the treatment is held when the serum ferritin level is about 50-100 mcg/L or below and until the serum ferritin level is equal to or of about 200-300 mcg/L or is above 200-300 mcg/L.

The present invention also pertains to the:
7. Use of the Compound of formula I for the preparation of a medicament for use in the treatment of hereditary hemochromatosis.
8. Use of the Compound of formula I for the preparation of a medicament for use in the treatment of an excess of iron in a patient having hereditary hemochromatosis.
9. Use of Compound I for the preparation of a medicament for use in patients having hereditary hemochromatosis and being ineligible for or inadequately treated by phlebotomy.
10. Use according to 7, 8 and 9 above wherein the hereditary hemochromatosis is due to the genetic homozygous mutation C282Y
11. use according to anyone of 7 to 10 above wherein the patient has a serum ferritin level equal or superior to 300 mcg/L.
12. use according to 11 above wherein the patient has a serum ferritin level equal or superior to 200 mcg/L.
13. use according to 11 above wherein the patient is a male patient,
14. use according to 12 above wherein the patient is a female patient.
15. use of Compound I for the manufacture of a medicament for use in any of the method according to 1 to 6 above.

The invention further pertains to a :
16. a pharmaceutical preparation comprising Compound I and at least one pharmaceutically acceptable salt thereof for any of the above mentioned use 7 to 15 and for use in any of the method described above 1 to 6.

The invention also pertains to a :
17. commercial package comprising Compound I, e.g. according to 16 above, together with instructions for use according to any of the use or method mentioned above.

The present invention further pertains to the use, method, pharmaceutical preparation and/or commercial package as described above wherein the hereditary hemochromatosis is juvenile hemochromatosis.

### Examples

### Example 1: safety and Efficacy of Compound I in patients with iron overload resulting from hereditary hemochromatosis.

This is an inter-patient dose escalation study exploring four dosages of Compound I (5, 10, 15, 20 mg/kg) given daily. Patients are sequentially assigned to each dose level starting with 5 mg/kg/day. The opening of each dose level is staggered by at least 4 weeks.
Patients are stratified by serum ferritin (at screening visit) into two strata:
Statrum 1: serum ferritin = 300-600 mcg/L and
Stratum 2: serum ferritin > 600 mcg/L to 1500 mcg/L .
Visits is scheduled every two weeks and treatment is continued for a total of 24 weeks.
The patients are adult hereditary hemochromatosis patients with iron overload.

### Inclusion criteria:

The inclusion criteria for the patients are the following:
- Age ≥ 18 years
- Male or female hemochromatosis patients homozygous for the C282Y mutation documented by molecular diagnostic testing.
- Serum ferritin value ≥ 300 mcg/L and ≤ 1500 mcg/L with a transferrin saturation ≥ 45%

### Dosing regimen:

The different dose regimens that are tested are the following 5 mg/kg PO per day, 10 mg/kg PO per day, 15 mg/kg PO per day, 20 mg/kg PO per day.

### Change in serum ferritin

If the serum ferritin level falls to <100 mcg/L at any visit, Compound I is held until the serum ferritin rises >300 mcg/L . Compound I is to be restarted at the same dose.

### Iron studies:

Serum ferritin, total iron, serum transferrin, and transferrin saturation is measured at screening and at every study visit. Labile plasma iron (LPI) is measured monthly.

These values are monitored as efficacy and safety parameters. In addition, the change in serum ferritin values obtained during the study serves as a secondary endpoint for efficacy analysis.

### Efficacy

The change from baseline in serum ferritin after 24 weeks of treatment are analyzed by performing an analysis of covariance ANCOVA, where the baseline serum ferritin measure is fitted as a continuous covariate. Adjusted means, standard deviation and 90% confidence intervals are presented and compared. A longitudinal analysis is also performed on the serum ferritin profile. Normalization of serum ferritin is specified as the first occurrence of reduction of serum ferritin < 100 mcg/L.

### Example 2: safety and Efficacy of Compound I in patients with iron overload resulting from hereditary hemochromatosis.

This is an inter-patient dose escalation study exploring four dosages of Compound I (5, 10, 15, 20 mg/kg) given daily. Patients are sequentially assigned to each dose level starting with 5 mg/kg/day. The opening of each dose level is staggered by at least 4 weeks.

Visits is scheduled every month and treatment is continued for a total of 24 weeks. The patients are adult hereditary hemochromatosis patients with iron overload.

### Inclusion criteria:

The inclusion criteria for the patients are the following:
- Age ≥ 18 years
- Male or female hemochromatosis patients homozygous for the C282Y mutation documented by molecular diagnostic testing.
- Serum ferritin value ≥ 300 mcg/L and ≤ 2000 mcg/L with a transferrin saturation ≥ 45%

### Dosing regimen:

The different dose regimens that are tested are the following 5 mg/kg PO per day, 10 mg/kg PO per day, 15 mg/kg PO per day, 20 mg/kg PO per day, if necessary dosage increments of 2.5 mg/kg/ PO per day are allowable.

### Change in serum ferritin : as in Example 1

Iron studies: as in Example 1

### Efficacy

The change from baseline in serum ferritin after 24 weeks of treatment are analyzed by performing an analysis of covariance ANCOVA, where the baseline serum ferritin measure is fitted as a continuous covariate. Adjusted means, standard deviation and 90% confidence intervals are presented and compared. A longitudinal analysis is also performed on the serum ferritin profile. Normalization of serum ferritin is specified as the first occurrence of reduction of serum ferritin < 100 mcg/L, alternatively of 50 mcg/L.

### Example 3: Compound I reduces iron overload in a murine model of juvenile hemochromatosis.

Introduction: Targeted disruption of the hemojuvelin gene in mice (HJV-/-) has recently been reported to cause markedly increased liver, pancreas and heart iron deposition, and act as a model for juvenile hemochromatosis (Niederkofler et al. 2005, Huang et al. 2005). Compound I, a novel tridentate oral iron chelator, is effective in the treatment of patients with transfusional iron overload and it has not been investigated in primary iron overload. We sought to examine spontaneous iron loading and the effect of Compound I upon the removal of iron in HJV-/- mice with iron overload.

**Methods:** Inductively-Coupled Plasma Optical Emission Spectrometry (ICP-OES, measurement of elemental iron) determinations in liver, heart, spleen and pancreas were performed at week 8 (study start), 14, 20 and 28 in HJV -/- and wild type (wt) mice (n=6-8), subsequent to MRI measurements in heart and liver. Starting at week 20 three groups of HJV-/- animals received daily 0 (vehicle), 30 or 100 mg/kg Compound I, control wt-mice remained untreated. Iron loading was observed up to 28 weeks (including vehicle group) and the effects of Compound I were assessed by comparing the 28-week groups. MRI R2* measurements were performed using a 4.7T MR imager. For the myocardial and hepatic R2* assessments single slice, ECG gated gradient echo images and single slice transversal gradient echo images were acquired, respectively.

**Results:** (see Figure 1 wherein Compound I is referred as "Deferasirox") Iron loading of the liver and the heart was 21- and 5-fold higher in HJV-/- than in wt mice.

Conspicuously, pancreas showed the most extreme difference in iron load (34-fold). Visual inspection of the time courses of iron loading between week 8 and 28 suggest a delayed loading of the heart and pancreas as compared to liver.

Effects of Compound I on organ iron load were modest at 30 mg/kg but quite pronounced in liver and heart at 100 mg/kg. This may seem contradictory to data from human studies, which show effects between 20 and 30 mg/kg in iron overload, but may be explained by the approximately 6-9 times lower exposure of Compound I in mice. At 100 mg/kg Compound I, liver iron was reduced by 63% and heart iron by 41% as compared to the (HJV-/-)-vehicle treated group. Pancreas showed a clear trend to lower levels at the 100 mg/kg dose. Contrary to liver, heart and pancreas, iron burden of the spleen was lower in HJV-/- as compared to wt mice. This behaviour is expected because the same (enhanced) iron efflux mechanism that allows iron to enter the circulation from enterocytes is also in place in reticuloendothelial cells.

MRI R2* values are more variable than organ iron concentrations determined by ICP-OES. However, the profiles of the time courses of **R2* of liver and heart** in HJV-/- mice were similar to the time courses of liver and heart iron as determined by ICP-OES, underlining the correlation between R2* values and organ iron.

**Conclusion:** Compound I significantly reduced liver and heart iron in the hemojuvelin knock out mouse and showed a trend to improvement of pancreatic iron load within 8 weeks of treatment.

## Claims

1. Use of the Compound of formula 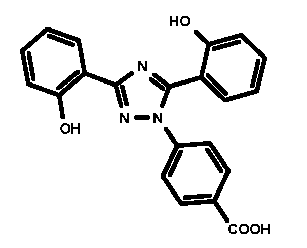 for the preparation of a medicament for use in the treatment of hereditary hemochromatosis.

2. Use of Compound I for the preparation of a medicament for the treatment of an excess of iron in a patient having hereditary hemochromatosis.

3. Use of Compound I for the preparation of a medicament for the treatment of an excess of iron in a patient having hereditary hemochromatosis and being ineligible for or inadequately treated by phlebotomy.

4. Use of Compound I for the preparation of a medicament for use in patients having hereditary hemochromatosis and being ineligible for or inadequately treated by phlebotomy.

5. Use according to anyone of claims 1 to 4 wherein the hereditary hemochromatosis is due to the genetic homozygous mutation C282Y or H63D.

6. Use according to any one of claims 1 to 5 wherein the patient has a serum ferritin level equal or superior to 300 mcg/L.

7. Use according to claim 6 wherein the patient has a serum ferritin level equal or superior to 200 mcg/L.

8. Use according to anyone of the preceding claims wherein the treatment is discontinued when the serum ferritin level is of about 100 mcg/L or below.

9. Use according to anyone of the preceding claims wherein the treatment is discontinued when the serum ferritin level is of about 50 mcg/L or below.

10. Use according to anyone of the preceding claims wherein the treatment is held when the serum ferritin level is of about 50-100 mcg/L or below and until the serum ferritin level is of equal or of about 200-300 mcg/L .

11. Method of treating hereditary hemochromatosis which causes iron overload in the human body comprising the administering to a patient in need of such a treatment a therapeutically effective amount of Compound I.

12. Method of treating iron overload resulting from hereditary hemochromatosis comprising the administering to a patient in need of such a treatment a therapeutically effective amount of Compound I.

13. Method according to claim 11 or 12 wherein the hereditary hemochromatosis is due to the mutation C282Y or H63D.

14. Method according to claim 11, 12 or 13 wherein Compound I is administered to a patient in need of such a treatment at a daily dose of about 5 to about 20 mg of Compound I /kg of body weight per day.

15. Method according to anyone of the preceding claims wherein the treatment is discontinued when the serum ferritin level is of about 100 mcg/L or below.

16. Method according to claim 15 wherein the treatment is discontinued when the serum ferritin level is of about 50 mcg/L or below.

17. Method according to anyone of the preceding claims wherein the treatment is held when the serum ferritin level is of about 50-100 mcg/L or below and until the serum ferritin level is of equal or of about 200-300 mcg/L .

18. Use according to claim 1 wherein the treatment of HH comprises:
(a) an induction phase wherein Compound of Formula I is administered at a daily dose of 5-20 mg/kg of body weight, followed by
(b) a maintenance phase wherein Compound of formula I is administered at a daily dose of 1 to 5 mg/kg of body weight
